(19) Europäisches Patentamt
European Patent Office
Office européen des brevets

(11) **EP 2 628 518 A1**

(12) **EUROPÄISCHE PATENTANMELDUNG**


(43) Veröffentlichungstag:
**21.08.2013 Patentblatt 2013/34**

(51) Int Cl.:
***B01D 19/04*** *(2006.01)* ***C10M 171/00*** *(2006.01)*
***C10B 55/00*** *(2006.01)* ***C10G 9/00*** *(2006.01)*

(21) Anmeldenummer: **13153955.3**

(22) Anmeldetag: **05.02.2013**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **20.02.2012 DE 102012202523**



(71) Anmelder: **Evonik Industries AG**
**45128 Essen (DE)**

(72) Erfinder:
- **Haensel, Rene**
  **46282 Dorsten (DE)**
- **Fiedel, Michael**
  **45133 Essen (DE)**
- **Ferenz, Michael**
  **45147 Essen (DE)**
- **Venzmer, Joachim**
  **45239 Essen (DE)**


(54) **Verwendung von selbstvernetzten Siloxanen zum Entschäumen von flüssigen Kohlenwasserstoffen**


(57) Die vorliegende Erfindung betrifft die Verwendung von Zusammensetzungen enthaltend die Komponenten A, ein Polymer erhältlich durch die Hydrosilylierung eines SiH-Funktionen und Vinyl-Funktionen aufweisenden Siloxane mit einer weiteren ungesättigten Verbindung, und D, Metallatome oder -ionen der Platingruppe als Entschäumer von Kohlenwasserstoffen sowie zur Unterdrückung bzw. Reduktion der Schaumbildung bzw. zur Schaumdestabilisierung.


EP 2 628 518 A1

## Beschreibung

Die vorliegende Erfindung betrifft die Verwendung von Zusammensetzungen enthaltend die Komponenten A, ein Polymer erhältlich durch die Hydrosilylierung eines SiH-Funktionen und Vinyl-Funktionen aufweisenden Siloxane mit einer weiteren ungesättigten Verbindung, und D, Metallatome oder -ionen der Platingruppe als Entschäumer von Kohlenwasserstoffen sowie zur Unterdrückung bzw. Reduktion der Schaumbildung bzw. zur Schaumdestabilisierung.

Die vorliegende Erfindung ist gerichtet auf die Verwendung einer Zusammensetzung aus verzweigten, organomodifizierten Polysiloxanen, mit einem Siliziumgehalt von weniger als 25 Gewichtsprozent, als Entschäumer von flüssigen Kohlenwasserstoffen bei hohen Temperaturen. Der Begriff flüssige Kohlenwasserstoffe soll auch solche organischen Systeme umfassen, die nicht vollständig, sondern zum überwiegenden Teil aus organischen Verbindungen bestehen.

Flüssige Kohlenwasserstoffe im Sinne der vorliegenden Erfindung können zur Anwendung kommen als z.B. Kraftübertragungsöle, wie Hydrauliköle, Getriebeöle oder Motorenöle. Hier kann beim Umpumpen oder fördern dieser organischen Systeme in Anwesenheit von Luft und gegebenenfalls von Wasser ein teilweiser starker Schaum entstehen. Bei Anwesenheit von weniger löslichen, polareren organischen Verbindungen, ist dieser Schaum oft beständig, da die weniger löslichen, polaren Verbindungen die Luft - Öldispersion, den Schaum, an der Grenzfäche stabilisieren können. Auch bei hohen Temperaturen z.B. > 100°C kann in solchen Anwendungen Schaum entstehen. Die Entwicklung von Schaum, welcher sich an der Oberfläche des organischen Systems beim Entweichen von Gas bildet, muss unterdrückt werden, da Schaum die rheologischen und chemischen Eigenschaften, z.B. durch Oxidation, der Systeme verändert und z.B. Schmierungs- und Kraftübertragungsprozesse und somit die Gebrauchszeiten von Öl und Maschinen negativ beeinträchtigen kann.

Weitere flüssige Kohlenwasserstoffe im Rahmen der vorliegenden Erfindung sind solche, die bei der destillativen Aufarbeitung von Rohöl unter atmosphärischen Druck und bei der Vakuumdestillation vorkommen. Teilweise werden die Destillationen z.B. in Raffinerien bei hohen Temperaturen durchgeführt. Schäume reduzieren dabei die Qualität der Trennung und beeinträchtigen die Kapazität der Trennapparatur. Weiterhin kommt es in Raffinerien in Crackprozessen z.B. beim Betrieb von sogenannten Visbreakem oder Cokem oft zu einer starken Schaumentwicklung unter extremen Temperaturbedingungen. Schäume verringern in diesen Anwendungen die Wirtschaftlichkeit des gesamten Prozesses.

Bei Prozessen, welche bei Raumtemperatur durchgeführt werden, sowie bei Temperaturen unter 100°C werden gemäß dem Stand der Technik Polyalkylsiloxane als Entschäumer eingesetzt, vorzugsweise Polydimethylsiloxane, in der Literatur oft PDMS abgekürzt, sogenannte Siliconöle. Aufgrund ihrer Unverträglichkeit, was sich häufig in Separation und damit reduzierter Langzeitwirkung äußert, und aufgrund des hohen Siliziumgehaltes, der oft unerwünscht ist, werden die PDMS bei diesen Bedingungen weitgehend durch organo-modifizierte Siloxane ersetzt.

Effektiv als Entschäumer bei Temperaturen unter 100°C sind z.B. herkömmliche Siliconpolyether-Copolymere. Diese enthalten einen oder mehrere Polyether, welche durch Anlagerung von Monomeren wie Propylenoxid und/oder Ethylenoxid, an Startalkohole, wie Allylalkohol, entstehen. Diese Produkte wirken schon ab einer Dosierung von 2 ppm.

Siliconpolyether-Copolymere wirken, da sie nur teilweise in dem zu entschäumenden Medium löslich sind und durch ihre Oberflächenaktivität in die Grenzfläche eintreten. Die Anpasung an die jeweiligen Bedingungen wird durch gezielte Struktur-Modifizierung der Siliconpolyether-Copolymere erreicht. Hierbei ist wesentlich, dass der Entschäumer eine niedrigere Oberflächenspannung als die zu entschäumende Flüssigkeit hat, um in die Grenzfäche Öl-Luft einzutreten, und dort zu spreiten was zur Zerstörung der Schaumblasen führt. Dieser bekannte Mechanismus wird von Denkov D.N., "Mechanismus of Action of Mixed Solid Liquid Antifoams-Stability of Oil Bridges in Foam Films", Langmuir, 1999, beschrieben. Aufgrund der notwendigen teilweisen Unlöslichkeit in den hier zu entschäumenden Kohlenwasserstoffen verbunden mit einer niedrigeren Oberflächenspannung, ist es erklärbar dass rein organisch basierte Entschäumer in der Industrie für diese Anwendungen aufgrund wesentlich geringerer Wirksamkeit, nur wenig Anwendung finden. Dies liegt ursächlich daran, dass rein organische Entschäumer diese beiden Eigenschaften in der Regel nicht zusammen aufweisen.

Als Beispiel zum Entschäumen von Kohlenwasserstoffen bei niedrigen Temperaturen dient z.B. die allgemeine Struktur aus DD 213945 A. Hier werden Schauminhibitoren für Schmieröle auf Mineralölbasis vorgeschlagen. Die vorgeschlagenen Verbindungen enthalten ein Polysiloxan-Grundgerüst mit seitenständigen Polyetherresten, welche mit Butylgruppen terminiert sind.

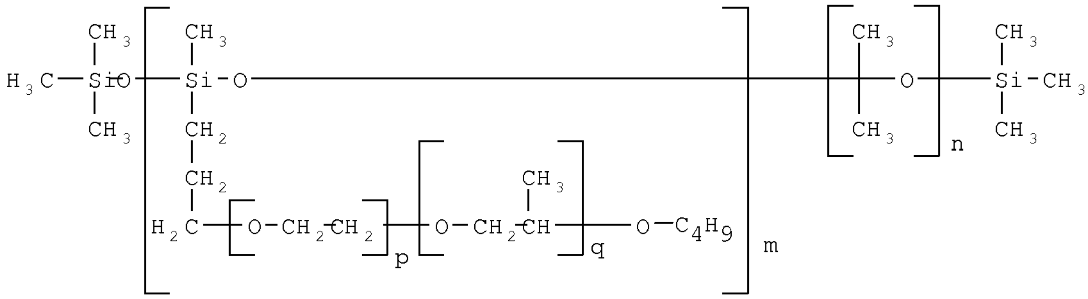

Ein weiteres Beispiel von organo modifizierten Siloxanen für die Entschäumung von Kohlenwasserstoffen z.B. Dieselkraftstoffe bei Raumtemperatur ist in der DE 4343235 C (US 5613988) beschrieben. Hier wird ein Verfahren zur Entschäumung von Dieselkraftstoff beschrieben, wobei organofunktionelle Polysiloxane der allgemeinen Formel

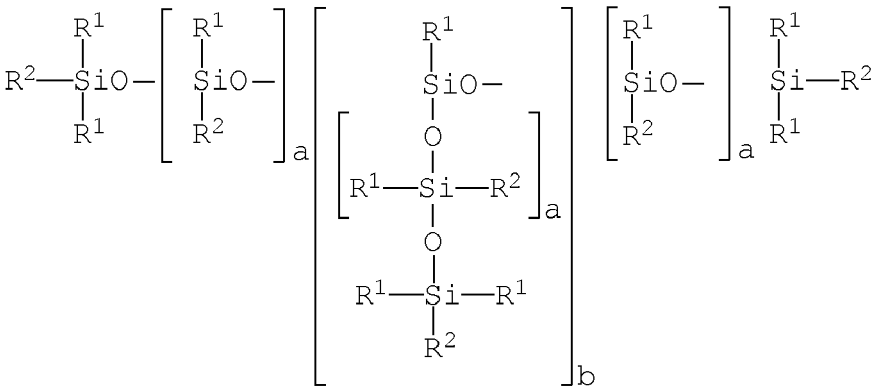

verwendet werden, wobei die Reste $R^1$ Alkyl- oder Arylreste sind, die Reste $R^2$ aus mehreren der nachfolgenden Verbindungsklassen ausgewählt sind: Butenderivate, Alkanolderivate, Polyether und Alkylreste.

US 5,542,690 und US 5,334,227 beschreiben die Verwendung von Organopolysiloxanen zur Entschäumung von Dieselkraftstoff. Dabei werden Polysiloxan-Terpolymere als Dieselentschäumer eingesetzt, die die Struktur $MD_xD^*_yD^{**}_zM$ aufweisen, mit M = $O_{0,5}Si(CH_3)_3$, D = $OSi(CH_3)_2$, D* = $OSi(CH_3)R$, mit R = einem Polyether, D** = OSi $(CH_3)R'$, mit R' = Phenolderivat und x+y+z = 35 bis 350, x/(y+z) = 3 bis 6 and y/z = 0,25 bis ca. 9,0. Die in US 5,334,227 beschriebenen Organopolysiloxane enthalten Polyether, die zu mehr als 75 % aus EthylenoxidEinheiten bestehen und keine Butylenoxid-Einheiten aufweisen.

Bei Kohlenwasserstoffsystemen, die bei höheren Temperaturen größer 100°C entschäumt werden müssen, wie es bei Motorenölen oder bei Destillationen z.B. in Raffinerien und bei Crackprozessen vorkommen kann, wirken die oben beschriebenen, herkömmlichen Organopolysiloxane nicht mehr oder nur bedeutend schlechter. Dies liegt möglicherweise an der durch die Temperaturerhöhung verbesserten Kompatibilität mit den zu entschäumenden Kohlenwasserstoffen. Bei noch höheren Temperaturen werden die Produkte vermutlich thermisch degradiert, die Abbauprodukte sind aufgrund des niedrigen Molekulargewichts wahrscheinlich nicht mehr wirksam.

Aus diesem Grunde ist es Stand der Technik zur Entschäumung von Kohlenwasserstoffen bei höheren Temperaturen für die verschiedenen Anwendungen hochmolekulare, hochviskose Poyldimethylsiloxane einzusetzen wie z.B: die Produktreihe von Dow Coming, in der unter dem Namen "Dow Corning 200 Fluid" Siliconöle mit verschiedenen Viskositäten vertrieben werden. Siliconöle wirken bereits in geringen Konzentrationen ab 2 ppm. Sie haben aber den bereits beschriebenen Nachteil, dass der hohe Siliziumgehalt, bei den überwiegend eingesetzten Polydimethylsiloxanen von ca. 38 Gew.-%, für viele Anwendungen nicht gewünscht ist. So führen siliziumhaltige Abbauprodukte in Raffinerien zur Vergiftung von Katalysatoren, die z.B. in nachgeschalteten Prozessen z.B. dem Hydrotreatment eingesetzt werden. Silikonöle haben weiterhin den Nachteil, dass sie das Luftabscheidevermögen von dispergierter Luft, sogenannter Mikroschaum, im Öl negativ beeinflussen. Dies ist für die Anwendung von Ölen als Schmierstoffe z.B. in Getrieben und Hydrauliksystemen ein Nachteil, da dispergierte Luft negative Eigenschaften auf die tribologischen Eigenschaften des Schmierstoffes hat. Die eindispergierten Luftblasen führen zu einem inhomogenen, dadurch instabilen, Schmierfilm, der z.B. leichter zwischen Zahnflanken ausbricht oder reißt. Die theoretischen Grundlagen zum Bildungsmechanismus dispergierter Luft in Öl und Oberflächenschaum aus gelöster und mechanisch eingearbeiteter Luft und die möglichen Auswirkungen im technischen Betrieb sind bekannt (Tourret & White: "Aeration and Foaming in Lubricating Oil Systems"; Aircraft Engng. 24 (1952), 122-130, 137). Daraus resultiert die Erkenntnis, dass für die Inhibierung des Oberflächenschaums und der dispergierten Luft gegenläufige Anforderungen bestehen. Oberflächenaktive Stoffe (wie z.B. Siliconöle), welche die Entstehung von Schaum inhibieren, wirken sich negativ auf das Luftabscheidevermögen (LAV) aus. Die organomodifizierten Siloxane der DD-A-213 945 verschlechtern bekanntermaßen das Luftabscheidevermögen weniger negativ als die Siliconöle, sie sind jedoch nur bei niedrigen Temperaturen geeignet.

Weiterhin wird in US20050109675 die Entschäumung nicht wässriger Systeme beschrieben. Die Zugabe von einem Siliconharz zu einem linearen Polydimethylsiloxan steigert die Wirksamkeit des linearen Silikonöls. Der thermische Abbau des linearen Silikonöls führt aber zu flüchtigen Crackprodukten und zur nachlassenden Wirksamkeit bei hohen Temperaturen.

Weiterhin werden in US 4,082,690 Mischungen aus Polydimethylsiloxanen mit Siliconharzen, sogenannte MQ-Harze, in einem Kohlenwasserstoff zur Entschäumung nicht wässriger Systeme beschrieben. Nachteil dieser silikonhaltigen Entschäumer ist jedoch der hohe Si-Gehalt von ca. 38 Gew.-%.

Aufgabe der vorliegenden Erfindung ist daher die Verwendung von Zusammensetzungen basierend auf organomodifizüerten Siloxanen, die als Entschäumer geeignet sind, die einen oder mehrere der Nachteile der siloxanbasierten Entschäumer des Standes der Technik nicht aufweisen, insbesondere eine deutliche Erniedrigung der Belastung der Produkte eines thermischen Verfahrens durch niedermolekulare siliziumhaltige Zersetzungsprodukte aufweisen.

<u>Beschreibung der Erfindung</u>

Überraschenderweise wurde gefunden, dass Zusammensetzungen enthaltend die Komponenten <u>A</u> und <u>D</u> und optional <u>B</u> und/oder <u>C</u>, wie nachfolgend definiert, diese Aufgabe lösen.

Gegenstand der vorliegenden Erfindung ist deshalb die Verwendung von Zusammensetzungen enthaltend die Komponenten <u>A</u> und <u>D</u> und optional <u>B</u> und/oder <u>C</u> wie in den Ansprüchen beschrieben.

Ein Vorteil der erfindungsgemäßen Verwendung ist, dass nach Anwendung thermischer Verfahren die Produkte einen deutlich reduzierten Siliziumgehalt gegenüber den bekannten Verfahren zur Entschäumung besitzen.

Ein weiterer Vorteil der erfindungsgemäßen Verwendung ist, dass die Zusammensetzungen enthaltend die Komponenten <u>A</u> und <u>D</u> und optional <u>B</u> und/oder <u>C</u> über einen sehr großen Temperaturbereich eingesetzt werden können.

Die Verwendung von Zusammensetzungen wird nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt vollständig zum Offenbarungsgehalt der vorliegenden Erfindung gehören. Werden nachfolgend oder vorhergehend Gehaltsangaben (ppm oder %) gemacht, so handelt es sich, wenn nicht anders angegeben, um Angaben in Gewichts-% oder Gewichts-ppm. Bei Zusammensetzungen beziehen sich die Gehaltsangaben wenn nicht anders angegeben auf die Gesamtzusammensetzung. Werden nachfolgend Mittelwerte angegeben, so handelt es sich, wenn nicht anders angegeben um Zahlenmittel. Werden Molmassen verwendet, handelt es sich, wenn nicht ausdrücklich anders vermerkt um gewichtsmittlere Molmassen Mw. Werden nachfolgend Messwerte angegeben, so wurden diese Messwerte, wenn nicht anders angegeben, bei einem Druck von 1013,25 hPa und einer Temperatur von 23°C ermittelt.

Die nachfolgenden Definitionen enthalten mitunter weitere Begriffe, die äquivalent und synonym zu dem definierten Begriff verwendet werden.

Unter dem Begriff "Molekulargewicht" wird, wenn nicht ausdrücklich anders vermerkt, im Sinne der Erfindung das Massenmittel der Molmassen, auch Molekulargewichtsmittel genannt, verstanden und als Mw abgekürzt und mit der Einheit g/mol verwendet.

Das Wortfragment "Poly" umfasst im Zusammenhang mit dieser Erfindung nicht nur ausschließlich Verbindungen mit zumindest 3 Wiederholungseinheiten eines oder mehrerer Monomere im Molekül, sondern insbesondere auch solche Zusammensetzungen von Verbindungen, die eine Molekulargewichtsverteilung aufweisen und dabei ein mittleres Molekulargewicht von mindestens 200 g/mol besitzen. Bei dieser Definition ist dem Umstand Rechnung getragen, dass es auf dem betrachteten Gebiet der Technik üblich ist, solche Verbindungen bereits als Polymere zu bezeichnen, auch wenn sie nicht einer Polymerdefinition analog OECD- oder REACH-Richtlinien zu genügen scheinen.

"Unter Anwendungsbedingungen flüssig" bedeutet, dass unter den jeweils gewählten Bedingungen eines Verfahrens, ggf. eines thermischen Verfahrens das zu entschäumende Medium flüssig ist. Hierfür kann es gegebenenfalls notwendig sein, dass das Medium geschmolzen werden muss, das heißt unter Normalbedingungen wie 25°C und 1013 mbar ist es fest und wird dann beim Erhitzen flüssig. Flüssig bedeutet in diesem Sinne, dass das Medium eine Viskosität von unter 120 Pa*s, bevorzugt unter oder bis zu 100 Pa*s besitzt. Das flüssige Medium kann gegebenenfalls auch Feststoffe enthalten.

Die hier wiedergegebenen Indexzahlen a, b, c, d, e, f, g, h, i, j, k, l, m, n, o, p, q und r, sowie die Wertbereiche der angegebenen Indizes können als Mittelwerte der möglichen statistischen Verteilung der tatsächlichen vorhandenen

Strukturen und/oder deren Mischungen verstanden werden. Dies gilt auch für als solche an sich exakt wiedergegebene Strukturformeln, wie beispielsweise für Formel (I), (II), (III) und (IV).

Die erfindungsgemäße Verwendung zeichnet sich dadurch aus, dass Zusammensetzungen zur Entschäumung von unter Anwendungsbedingungen flüssigen Kohlenwasserstoffen verwendet werden, wobei die verwendungsgemäßen Zusammensetzungen die Komponenten A und D enthalten, mit

A enthaltend ein Polymer erhältlich durch Umsetzung im Sinne einer Hydrosilylierung von Verbindungen der Formel (I)

$$M_a M^v_b M^H_c D_d D^H_e D^v_f T_g Q_h \qquad \text{Formel (I)}$$

mit

M = $[R^1_3SiO_{1/2}]$
$M^V$ = $[R^3R^1_2SiO_{1/2}]$
$M^H$ = $[R^1_2SiHO_{1/2}]$
D = $[R^1_2SiO_{2/2}]$
$D^H$ = $[R^1SiHO_{2/2}]$
$D^V$ = $[R^3R^1SiO_{2/2}]$
T = $[R^1SiO_{3/2}]$
Q = $[SiO_{4/2}]$
a = 0 bis 42, bevorzugt 0 bis 22, insbesondere bevorzugt größer 0 bis 2,
b = 0 bis 42, bevorzugt gleich oder größer 1 bis 22, insbesondere bevorzugt größer 1 bis kleiner 2,
c = 0 bis 42, bevorzugt 0 bis 22, insbesondere bevorzugt 0,
d = 5 bis 600, bevorzugt 10 bis 400, mehr bevorzugt 20 bis 300, insbesondere bevorzugt 50 bis 200,
e = 0 bis 50, bevorzugt größer 0 bis 25, mehr bevorzugt 0,5 bis 10, insbesondere bevorzugt 0,7 bis 1,5,
f = 0 bis 50, bevorzugt 0 bis 25, insbesondere bevorzugt 0,
g = 0 bis 20, bevorzugt größer 0 bis 10, insbesondere bevorzugt 1 bis 5,
h = 0 bis 20, bevorzugt 0 bis 10, insbesondere bevorzugt 0,

mit der Maßgabe, dass folgende Bedingungen erfüllt sind
a + b + c größer oder gleich 2,
b + f größer 0, bevorzugt größer oder gleich 1,2 und kleiner 2,
c + e größer 0, bevorzugt größer 0,8, mehr bevorzugt größer 1 bis 8, insbesondere bevorzugt von 1,2 bis kleiner 6 und 0,24*(a+b+c+d+e+f+g) größer (c+e)

$R^1$ unabhängig voneinander gleiche oder verschiedene Alkylreste mit 1 bis 30 Kohlenstoffatomen, oder gleiche oder verschiedene Arylreste mit 6 bis 30 Kohlenstoffatomen oder-OH oder-$OR^2$,
bevorzugt Methyl, Phenyl, -OH oder-$OR^2$,
insbesondere Methyl oder Phenyl,
$R^2$ unabhängig voneinander gleiche oder verschiedene Alkylreste mit 1 bis 12 Kohlenstoffatomen, oder gleiche oder verschiedene Arylreste mit 6 bis 12 Kohlenstoffatomen, bevorzugt Methyl, Ethyl, n-Propyl, Isopropyl, n-Butyl, sek-Butyl, iso-Butyl, Phenyl,
insbesondere Methyl und Ethyl,
$R^3$ unabhängig voneinander gleiche oder verschiedene organische Reste mit einer terminalen Doppelbindung oder einer terminalen oder inneren Dreifachbindung,
bevorzugt organische Reste mit einer terminalen Doppelbindung,
insbesondere Vinyl (d.h. $-CH=CH_2$) oder Allyl (d.h. $-CH_2CH=CH_2$),
mit Verbindungen der Formel (I) und/oder mit anderen Verbindungen C, die eine C-C-Mehrfachbindung aufweisen, und nicht Formel (I) entsprechen,
und
D Metallatomen und/oder -ionen der Platingruppe, bevorzugt Platin-, Rhodium- und Rutheniumatomen, insbesondere Platinatomen.

Die Verbindungen der Formel (I) können als selbstvernetzende Siloxane bezeichnet werden. Sie sind dadurch gekennzeichnet, dass sie neben SiH-Funktionen der Hydrosilylierung zugängliche Mehrfachbindungen aufweisen und somit zwei oder mehr Verbindungen der Formel (I) miteinander im Rahmen einer Hydrosilylierung reagieren können.

Es können erfindungsgemäß Zusammensetzungen verwendet werden, die Komponente A ausschließlich oder neben weiteren Polymeren ein Polymer enthalten, welches erhältlich ist durch Umsetzung im Sinne einer Hydrosilylierung

von Verbindungen der Formel (I) und Verbindungen der Formel (II)

$$M_i\, M^H_j\, D_k\, D^H_l\, T_m\, Q_n \qquad \text{Formel (II)}$$

mit

- i = 0 bis 34, bevorzugt 0 bis 18, insbesondere bevorzugt größer 0 bis 2,
- j = 0 bis 34, bevorzugt 0 bis 18, insbesondere bevorzugt größer 0 bis 2,
- k = 5 bis 600, bevorzugt 10 bis 400, mehr bevorzugt 20 bis 200, insbesondere bevorzugt 50 bis 150,
- l = 0 bis 50, bevorzugt größer 0 bis 35, mehr bevorzugt 1 bis 26, insbesondere bevorzugt größer 1 bis 10,
- m = 0 bis 16, bevorzugt 0 bis 8, insbesondere 0,
- n = 0 bis 16, bevorzugt 0 bis 8, insbesondere 0,

und den Bedingungen
i + j größer oder gleich 2 und
j + l größer oder gleich 2.

Vorzugsweise weisen die verwendungsgemäßen Zusammensetzungen eine Komponente A auf, die ein Polymer enthält, erhältlich durch Umsetzung im Sinne einer Hydrosilylierung von Verbindungen der Formel (I) mit einer oder mehreren ungesättigten Verbindungen C. Bevorzugt weisen die erfindungsgemäßen Zusammensetzungen eine Komponente A auf, die ein Polymer enthält, erhältlich durch Umsetzung im Sinne einer Hydrosilylierung von Verbindungen der Formel (I) mit einer Verbindung der Formel (II) und einer oder mehreren ungesättigten Verbindungen C.

Es kann vorteilhaft sein, wenn die verwendungsgemäßen Zusammensetzungen eine Komponente B enthalten, erhältlich durch Umsetzung im Sinne einer Hydrosilylierung von Verbindungen der Formel (II), wie oben definiert und ungesättigten Verbindungen C.

Die verwendungsgemäßen Zusammensetzungen können eine oder mehrere Verbindungen C enthalten, diese können nachträglich zur Zusammensetzung zugegeben werden oder als nicht umgesetzter Reaktand bei der Herstellung der Zusammensetzung in der Zusammensetzung verbleiben.

Die oben genannten Verbindungen C sind vorzugsweise Olefine oder Polyether, die eine oder mehrere Kohlenstoff-Kohlenstoff-Mehrfachbindungen aufweisen, bevorzugt Polyether, die eine oder mehrere Kohlenstoff-Kohlenstoff-Mehrfachbindungen aufweisen.

Bevorzugte Olefine sind Olefine mit endständigen Doppelbindungen, z. B. alpha-Olefine, alpha,omega-Olefine, allylgruppentragende Mono- und Polyole oder Allylgruppen tragende Aromaten. Insbesondere bevorzugte Olefine sind Ethen, Ethin, Propen, 1-Buten, 1-Hexen, 1-Dodecen, 1-Hexadecen, 1,3-Butadien, 1,7-Octadien, 1,9-Decadien, Styrol, Eugenol, Allylphenol, Undecylensäuremethylester, Allylalkohol, Allyloxyethanol, 1-Hexen-5-ol, Allyamin, Propagylalkohol, Propagylchlorid, Propagylamin oder 1,4-Butindiol.

Bevorzugte Polyether mit einer oder mehreren Mehrfachbindungen sind zum Beispiel allylfunktionelle Polyether oder 1,4-Butindiol gestartete Polyether. Besonders bevorzugte Polyether, die Kohlenstoff-Kohlenstoff-Mehrfachbindungen aufweisen, sind vorzugsweise solche der Formel (III),

$$CH_2{=}CHCH_2O(C_2H_4O)_o(C_2H_3(CH_3)O)_p(C_2H_3(C_2H_5)O)_q(C_2H_3(Ph)O)_rR^4 \qquad \text{Formel (III)}$$

mit
$R^4$ unabhängig voneinander gleiche oder verschiedene organische Reste, die keine der Hydrosilylierung zugängliche Mehrfachbindung tragen,
bevorzugt Wasserstoff, Alkylreste oder Carboxyreste,
besonders bevorzugt Wasserstoff, Methyl, Butyl oder Acetyl,
insbesondere bevorzugt Wasserstoff,
o = 0 bis 200, bevorzugt größer 0 bis 150, besonders bevorzugt größer oder gleich 2 bis zu 120, insbesondere bevorzugt gleich oder größer 3 bis zu 90,
p = 0 bis 200, bevorzugt größer 0 bis 150, besonders bevorzugt größer oder gleich 2 bis zu 100, insbesondere bevorzugt gleich oder größer 3 bis zu 80,
q = 0 oder größer 0 bis 100, bevorzugt 0 oder größer 0 bis 30, besonders bevorzugt 0 oder größer 0 bis 1, insbesondere 0,
r = 0 oder größer 0 bis 100, bevorzugt 0 oder größer 0 bis 30, besonders bevorzugt 0 oder größer 0 bis 1, insbesondere 0,
und den Bedingungen
o + p + q + r größer 3, vorzugsweise ist o größer 0, mehr bevorzugt ist o größer p + q + r; besonders bevorzugt o größer

p, insbesondere bevorzugt o größer 1,5 * p und wenn q + r kleiner 2, dann ist o größer 4 * p,
im Falle von p = 0, ist o mindestens 4, bevorzugt mindestens 8,
im Falle von p = 0 und q + r gleich oder größer 2 ist o mindestens 2 * (q + r).

Ganz besonders bevorzugte Polyether sind zum Beispiel:

$CH_2=CHCH_2O(C_2H_4O)_{20}(C_2H_3(CH_3)O)_{4,5}H$
$CH_2=CHCH_2O(C_2H_4O)_{25}(C_2H_3(CH_3)\ O)_{4,5}H$
$CH_2=CHCH_2O(C_2H_4O)_8H$
$CH_2=CHCH_2O(C_2H_4O)_{20}(C_2H_3(CH_3)\ O)_{4,5}Me$
$CH_2=CHCH_2O(C_2H_4O)_{26}(C_2H_3(CH_3)\ O)_{4,5}Me$
$CH_2=CHCH_2O(C_2H_4O)_8Me$
$CH_2=CHCH_2O(C_2H_4O)_{20}(C_2H_3(CH_3)O)_{4,5}$Acetyl
$CH_2=CHCH_2O(C_2H_4O)_{26}(C_2H_3(CH_3)O)_{4,5}$ Acetyl
$CH_2=CHCH_2O(C_2H_4O)_8$ Acetyl
$CH_2=CHCH_2O(C_2H_4O)_8(C_2H_3(Ph)O)_3H$
$CH_2=CHCH_2O(C_2H_4O)_8(C_2H_3(Ph)O)_2H$

Polyether, die eine oder mehrere Kohlenstoff-Kohlenstoff-Mehrfachbindungen aufweisen, sind kommerziell erhältlich, z.B. unter den Markennamen Pluriol® (BASF) oder Polyglycol AM® (Clariant).

Besonders bevorzugt werden Zusammensetzungen erfindungsgemäß verwendet, enthaltend die Komponente A ihrerseits enthaltend Verbindungen der Formel (I) mit

a = 0 bis 2,
b = größer 1 bis 22,
c = 0 bis 22,
d = 50 bis 400,
e = 0 bis 1,5,
f, g, h = 0 und den Definitionen der Reste wie oben angegeben und Verbindungen der Formel (II), mit
i = 1 bis 2,
j = 0 bis 1,
k = 50 bis 150,
l = größer 1 bis 26,

Komponente B,

Verbindungen C der Formel (III) mit
wenn q + r kleiner 2, dann ist o größer 4 * p,
im Falle von p = 0, ist o mindestens 8
im Falle von p = 0 und q + r gleich oder größer 2 ist o mindestens 2 * (q + r), und die Komponente D in Form von Platinatomen und/oder Platinionen.

Ohne an diese Theorie gebunden zu sein, sind die verwendungsgemäßen Zusammensetzungen als Entschäumer in hydrophoben Systemen vermutlich aufgrund ihrer mangelnden Mischbarkeit mit dem zu entschäumenden Medium aktiv (ref Venzmer et al. in Bartz, W. J. (Ed.): 5th International Colloquium Fuels, TAE Esslingen, January 12-13, 2005, 483-488,). Deshalb können hydrophile Substituenten in den Komponenten A, B und D, sowie in Verbindung C wie polyoxyethylenreiche Molekülteile, Polyethylenglycolartige Molekülteile und/oder Hydroxygruppen hier einen besonderen Beitrag leisten und sind damit bervorzugt.

Vorzugsweise weisen die verwendungsgemäßen Zusammensetzungen die Komponente A mit einem Anteil von 1 bis 90 Gew.-%, vorzugsweise größer 1 bis 30 Gew.-%, bevorzugt 1 bis 15 Gew.-%,
die Komponente B mit einem Anteil von 0 bis 70 Gew. %, vorzugsweise größer 0 bis 40 Gew.-%. bevorzugt 1 bis 30 Gew.-%,
die Verbindungen C mit einem Anteil von 0 bis 95 Gew.%, vorzugsweise 5 bis 90 Gew.-% bevorzugt 10 bis kleiner 90 Gew.-% und
die Komponente D mit einem Anteil von größer 0 bis 50 Gew.-ppm,
jeweils bezogen auf die Masse der Gesamtzusammensetzung, auf.

Bevorzugt weisen die verwendungsgemäßen Zusammensetzungen
die Komponente A mit einem Anteil von 1 bis 15 Gew.%,
die Komponente B mit einem Anteil von 1 bis 30 Gew.%,
die Verbindungen C mit einem Anteil von 10 bis kleiner 90 Gew.-% und
die Komponente D mit einem Anteil von größer 0 bis 50 Gew.-ppm,

jeweils bezogen auf die Masse der Gesamtzusammensetzung, auf.

In den verwendungsgemäßen Zusammensetzungen liegt das Polymer der Komponente A zu mehr als 90 Gew.-% bezogen auf die Komponenten A mit einer gewichtsmittleren Molmasse von weniger als 2500000 g/mol vor.

In den verwendungsgemäßen Zusammensetzungen liegt die Komponente B zu mehr als 90 Gew.-% bezogen auf die Komponente B mit einer gewichtsmittleren Molmasse von bis zu 1000000 g/mol vor. Eine solche Komponente B ist vorzugsweise in der Zusammensetzung mit kleiner als 5 Gew.-%, bezogen auf die Gesamtzusammensetzung vorhanden.

Bevorzugte verwendungsgemäße Zusammensetzungen zeichnen sich dadurch aus, dass die Komponente

A zu mehr als 90 Gew.-% bezogen auf die Komponenten A Polymere mit einer gewichtsmittleren Molmasse von weniger als 2500000 g/mol aufweist und die Komponente

B zu mehr als 90 Gew.-% bezogen auf die Komponente B mit einer gewichtsmittleren Molmasse von bis zu 1000000 g/mol vorliegen und die Komponente B in der Zusammensetzung mit kleiner als 5 Gew.%, bezogen auf die Gesamtzusammensetzung vorhanden ist.

Die Zusammensetzungen der erfindungsgemäßen Verwendung sind vorzugsweise bei 20°C und 1013 mbar flüssig. Flüssig im Sinne der Erfindung sind Substanzen, homogene und/oder heterogene Mischungen, die bei Raumtemperatur, bevorzugt bei 20°C und Normaldruck (1013 mbar) eine Viskosität von kleiner 120 Pa*s, bevorzugt kleiner 100 Pa*s und besonders bevorzugt kleiner 10 Pa*s besitzen. Bevorzugte Zusammensetzungen weisen demgemäß vorzugsweise eine entsprechende Viskosität, bestimmt wie in den Beispielen angegeben, auf.

Die Zusammensetzungen der erfindungsgemäßen Verwendung zeichnen sich vorzugsweise dadurch aus, dass sie einen Gehalt von weniger als 25 Gew.%, bevorzugt weniger als 20 Gew.-%, besonders bevorzugt weniger als 15%, und ganz besonders bevorzugt von 0,01 bis 10 Gew.-% Silizium bezogen auf die Summe der Massen der Komponenten A, B und D und Verbindung C der Zusammensetzung aufweisen.

Der Gehalt an Metallatomen und/oder-ionen der Platingruppe in den Zusammensetzungen der erfindungsgemäßen Verwendung beträgt vorzugsweise größer 0 bis 50 wppm (Massen-ppm), bevorzugt 1 bis 40 wppm, besonders bevorzugt 3 bis 30 wppm, ganz besonders bevorzugt 5 bis 20 wppm und insbesondere bevorzugt 8 bis 10 wppm bezogen auf die Gesamtmasse der Zusammensetzung. Bevorzugt liegen in der Zusammensetzung Platin, Rhuthenium und/oder Rhodium in diesen Konzentrationen vor.

Die verwendungsgemäßen Zusammensetzungen können gegebenenfalls weitere Zusätze enthalten. Bevorzugte Zusätze sind aliphatische und/oder aromatische Öle und Lösemittel.

Bevorzugte Lösemittel sind z. B. Alkohole und aliphatische Kohlenwasserstoffe. Bevorzugte Alkohole können z.B. Methanol, Ethanol, Ethylenglykol, n-Propanol, iso-Propanol, 1,2-Propylenglykol, 1,3-Propylenglykol, n-Butanol, 2-Butano und tert.-Butanol sein. Bevorzugte Kohlenwasserstoffe sind insbesondere Kohlenwasserstoffe mit einem Siedepunkt bei Normaldruck (1013 mbar) von kleiner 250 °C.

Die Zusammensetzungen der erfindungsgemäßen Verwendung enthalten gegebenenfalls Verbindungen charakterisiert durch die Teilstruktur der Formel (V).

$$CH_3\text{-}CH{=}CH2\text{ -} \qquad \text{Formel (V)}$$

Bevorzugte Verbindungen enthaltend die Teilstruktur der Formel (V) sind Polyether der Formel (IV)

$$CH_3\text{-}CH{=}CH_2\text{-}O(C_2H_4O)_o(C_2H_3(CH_3)O)_p(C_2H_3(C_2H_5)O)_q(C_2H_3(Ph)O)_rR^4 \qquad \text{Formel (IV)}$$

wobei die Indices und der Rest $R^4$ wie bei Formel (III) definiert sind. Die dort für Formel (III) angegebenen Vorzugsbereiche gelten in gleicher Weise auch für die Verbindungen der Formel (IV).

Die Verbindungen der Formeln (IV) und/oder (V) können der verwendungsgemäßen Zusammensetzung zusätzlich hinzugefügt werden oder z. B. durch Umlagerungen an C-C-Mehrfachbindungen im Verlauf der Herstellung der Zusammensetzung, insbesondere bei der Umsetzung unter hydrosilylierenden Bedingungen entstehen.

Der Anteil an Verbindungen, die eine Teilstruktur der Formel (V) aufweisen, vorzugsweise Verbindungen der Formel (IV), an der erfindungsgemäßen Zusammensetzung beträgt vorzugsweise von 0,0001 bis 25 Gew.-%, bevorzugt von 0,01 bis 20 Gew.%.

Es kann vorteilhaft sein, wenn die verwendungsgemäßen Zusammensetzung keine Verbindungen, die eine Teilstruktur der Formel (V) aufweisen, aufweist bzw. der Anteil so gering ist, dass er sich nicht analytisch nachweisen lässt.

Die verschiedenen Fragmente in den Formeln (I), (II), (III) und (IV) können statistisch verteilt sein. Statistische Verteilungen können blockweise aufgebaut sein mit einer beliebigen Anzahl an Blöcken und einer beliebigen Sequenz oder sie können einer randomisierten Verteilung unterliegen, sie können auch alternierend aufgebaut sein oder auch über die Kette einen Gradienten bilden, insbesondere können sie auch alle Mischformen bilden, bei denen gegebenenfalls

Gruppen unterschiedlicher Verteilungen aufeinanderfolgen können.

Die Zusammensetzungen der erfindungsgemäßen Verwendung können in an sich bekannter Art und Weise durch Hydrosilylierung gewonnen werden. Als Katalysatoren für die Hydrosilylierungsreaktion eignen sich prinzipiell Platinverbindungen wie beispielsweise Hexachloroplatinsäure, cis-Platin, Bis-(cycloocten)Platindichlorid, carbo-Platin, Platin(0)-(divinyttetramethytdisitoxan)-Komptexe, sogenannte Karstedt-Katalysatoren, oder auch mit unterschiedlichen Olefinen komplexierte Platin(0)-Komplexe. Des Weiteren eignen sich prinzipiell Rhodium-, Iridium- und Rutheniumverbindungen, wie beispielsweise Tris(triphenylphosphin)-Rhodium(I)chlorid oder Tris(triphenylphosphin)-Rhuthenium(II) dichlorid. Im Sinne des Verfahrens bevorzugte Katalysatoren sind Platin(0)-Komplexe, insbesondere bevorzugt sind Karstedt-Katalysatoren oder sogenannte WK-Katalysatoren, die gemäß EP1520870 hergestellt werden.

Im Verlauf einer Hydrosilylierung sind eine Reihe von Nebenreaktionen möglich. Zum Beispiel ist dem Fachmann bekannt, dass es durch die dehydrogenative Anlagerung von Alkoholfunktionen an die SiH Bindung zur Bildung von SiOC - Bindungen kommen kann. Auch kann ein geringer Restwasseranteil zum Abbau von SiH Funktionen führen. Ferner sind Umlagerungen an C-C-Mehrfachbindungen bekannt.

Vorzugsweise werden die Zusammensetzungen verwendet zur Entschäumung von Dieselkraftstoffen, Schmierölen in Getrieben, Motoren oder Hydrauliksystemen, sowie bei thermischen Verfahren. Die thermischen Verfahren können die zu entschäumende Komponente oder Komponenten in ihrer strukturellen Identität erhalten oder aber auch in ihrer Identität verändern. Derartige Veränderungen können destruktiver, also z.B. zersetzend und/oder konstruktiver, z.B. im Sinne der Bildung neuer ggf. erwünschter Substanzen, Natur sein. Bevorzugte thermische Verfahren sind z.B. Destillationen oder Crackprozesse. Die thermischen Verfahren können sowohl bei Normaldruck, als auch bei Unterdruck (Vakuum) oder Überdruck durchgeführt werden. Besonders bevorzugte Verwendungen sind die Anwendung bei Destillationen, sowohl im Vakuum als auch bei Atmosphärendruck. Weiterhin bevorzugt sind Anwendungen bei Crackprozessen.

Die Zusammensetzungen können vorteilhafterweise bei Temperaturen von 80 bis 200°C, bevorzugt von 100°C bis 180°C, insbesondere bevorzugt von 120 bis 160°C zur Anwendung kommen. Diese Temperaturen werden vorzugsweise bei Destillationen angewendet.

Die Zusammensetzungen können weiterhin vorteilhafterweise bei Temperaturen von bis zu 600°C, bevorzugt von 150 bis 500°C, mehr bevorzugt von 200 bis 450°C, weiter mehr bevorzugt von 250 bis 400°C, insbesondere von 300 bis 350°C verwendet werden. Diese Temperaturen werden vorzugsweise bei Crackprozessen angewendet. Crackprozesse können z.B. in Delayed Cokem oder Visbreakern durchgeführt werden.

Bevorzugt werden die Zusammensetzungen bei Destillationen und/oder Crackprozessen in Raffinerien verwendet.

Die Zusammensetzungen können weiterhin vorteilhafterweise zum Entschäumen von unter Anwendungsbedingungen flüssigen Kohlenwasserstoffen wie Schmierstoffen und Kraftübertragungsölen, eingesetzt werden. Die Temperaturen können hierbei deutlich über 100°C liegen.

Die Zusammensetzungen können den zu entschäumenden Kohlenwasserstoffen direkt, d.h. ohne weitere Zusätze, als auch in einer Mischung mit aliphatischen oder aromatischen Lösungsmittel zugesetzt werden. Bevorzugt werden die erfindungsgemäßen Zusammensetzungen in einer Mischung mit aliphatischen oder aromatischen Lösungsmitteln oder deren Mischungen eingesetzt.

Die Zusammensetzungen können zur erfindungsgemäßen Verwendung den zu entschäumenden Kohlenwasserstoffen kontinuierlich oder absatzweise zugesetzt werden. Im Falle von Destillationen werden die Zusammensetzungen bevorzugt kontinuierlich zugesetzt. Die Zugabe der Zusammensetzungen kann auch durch die Anwendung von Messarmaturen zur Bestimmung der Schaummenge bzw. Höhe determiniert werden. Steigt der Schaum über einen bestimmten Grenzwert an, wird die Zugabe des Entschäumers ausgelöst. Diese Zugabe kann sowohl in die Flüssigphase des zu entschäumenden Kohlenwasserstoffes erfolgen, als auch direkt auf den Schaum. Bei einer Zugabe auf den Schaum kann die Zugabe in unterschiedlichen Verteilungen erfolgen, bevorzugt ist eine Dispersion in der vorliegenden Atmosphäre, besonders bevorzugt in einem Sprühverfahren. Besonders bevorzugt ist die Zugabe der Zusammensetzungen durch Sprühen auf den Schaum im Falle von Crackprozessen.

Die vorliegende Erfindung wird an Hand der Abbildung Fig. 1 näher erläutert, ohne dass die Erfindung, deren Anwendungsbreite sich aus der gesamten Beschreibung und den Ansprüchen ergibt, auf die in der Abbildung genannten Ausführungsform beschränkt sein soll. Fig. 1 zeigt einen schematischen Aufbau einer Apparatur zur Durchführung von Entschäumungsexperimenten, dem sogenannten Frittentest.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Beispiele

Allgemeine Methoden und Materialien

Viskosität:

Bestimmung der Viskosität mittels eines Spindelviskosimeter Typ Brookfield LV-DV-I+ Brookfield-Viskosimeter sind Rotationsviskosimeter mit definierten Spindelsätzen als Rotationskörper. Bei den verwendeten Rotationskörpern handelte es sich um einen LV-Spindelsatz. Aufgrund der Temperaturabhängigkeit der Viskosität wurde die Temperaturen von Viskosimeter und Messflüssigkeit während der Messung auf +/- 0,5 °C bei 20 °C genau konstant gehalten. Weitere verwendete Materialien neben dem LV-Spindelsatz waren ein thermostatisierbares Wasserbad, ein Thermometer 0-100°C (Skalenteile 1°C bzw. kleiner) und
ein Zeitmessgerät (Skalenwerte nicht größer als 0,1 Sekunden). Zur Messung wurden 100 ml der Probe in eine Weithalsflasche gefüllt; temperiert und luftblasenfrei vermessen, nachdem eine vorherige Kalibrierung erfolgt war. Zur Bestimmung der Viskosität wurde das Viskosimeter so zur Probe positioniert, dass die Spindel bis zur Markierung im Produkt eintaucht. Die Messung wird mit Hilfe der Starttaste ausgelöst, wobei darauf geachtet wurde, dass die Messung im günstigen Messbereich von 50% (+/- 20%) des maximal messbaren Drehmoments erfolgte. Das Ergebnis der Messung wurde am Display des Viskosimeters in mPas ausgegeben, wobei die Division durch die Dichte (g/ml) die Viskosität in $mm^2/s$ liefert.

Bestimmung des SiH-Gehaltes

Die Bestimmungen der SiH-Werte der eingesetzten Wasserstoffsiloxane aber auch die der Reaktionsmatrices erfolgen jeweils gasvolumetrisch durch die Natriumbutylat induzierte Zersetzung aliquot eingewogener Probemengen an einer Gasbürette. Eingesetzt in die allgemeine Gasgleichung gestatten die gemessenen Wasserstoffvolumina die Gehaltsbestimmung aktiver SiH-Funktionen in den Edukten aber auch in den Reaktionsansätzen und erlauben so die Umsatzkontrolle.

Bestimmung der Molmassenverteilungen:

Die gelpermeationschromatographischen Analysen (GPC) erfolgten mit einem Gerät Typ 1100 der Firma Hewlett-Packard unter Verwendung einer SDV- Säulenkombination (1000/10000 Å, je 65cm, Innendurchmesser 0,8 cm, Temperatur 30°C), THF als mobiler Phase mit einer Flussrate von 1ml/min und einem RI-Detektor (Hewlett-Packard). Die Kalibrierung des Systems erfolgte gegen einen Polystyrolstandard im Bereich von 162 - 2 520 000 g/mol.

Die GPC Analysen wurden bei unterschiedlichen Temperaturen durchgeführt. Aus dem linearen Bereich der Messungen wurden die jeweiligen Messwerte entnommen und gegen den ausgewählten Standard die Molmassen berechnet.

| Material | | Lieferant |
|---|---|---|
| Decamethylcyclopentasiloxan | $D_5$ | ABCR (KatNr. AB111012) |
| Trifluormethan-sulfonsäure | TFMSA | Aldrich (Kat.-Nr. 347817) |
| Solvesso 150 (aliphatisches Lösemittel) | | Exxon Mobil Corporation |
| Divinyltetrammethyldisiloxan | | ABCR (Kat.Nr. AB121873) |
| Polymethylphenylsiloxans (500 cSt) | | ABCR, Artikel PMM-0025 |
| Allylpolyether 1 | Iodzahl = 18,5 g/100g, OHZ = 44 mg KOH/g, 23 Gew.% PO | |

Die eingesetzten Karstedt-Lösungen sind Platin(0)-Divinyltetramethyldisiloxankomplexe in Decamethylcyclopentasiloxan in der Konzentration von 0,1 Gew. % Platin (erhältlich bei Umicore mit 21,37 Gew. % Platin, welches durch Verdünnen mit Decamethylcyclopentasiloxan auf 0,1 Gew. % Pt eingestellt wird). Die in den folgenden Beispielen angegebenen Dosierungen des Katalysators beziehen sich auf die Massensumme der Einwaagen der Reaktionskomponenten der Hydrosilylierung, zugesetzte Lösemittel werden in dieser Berechnung nicht berücksichtigt.

Beispiel 1: Synthesebeispiele

S1 (Ausgangmaterial für Synthesebeispiel S3): Herstellung eines Equilibrates gemäß Formel (I) In einem Mehrhalskolben, ausgestattet mit Rührvorrichtung, Stickstoffzuleitung und Rückflusskühler werden 3,1 g Divinyltetramethyldisiloxan, 0,98 g eines mehrfach mittelständigen Wasserstoffsiloxans (15,7 eq/kg SiH), 194,7 g $D_5$ und 0,12 ml Trifluormethan-sulfonsäure vorgelegt und bei Raumtemperatur 24 Stunden gerührt. Nach vollständiger Equilibrierung wird durch Zugabe von 4,0 g Natriumhydrogencarbonat binnen 2 Stunden neutralisiert und nachfolgend filtriert. Es wurde ein farbloses, klares Silikonequilibrat erhalten.

S2: Herstellung eines Equilibrates gemäß Formel (II), $R^1$: Me und Phenyl)

In einem Mehrhalskolben, ausgestattet mit Rührvorrichtung, Stickstoffzuleitung und Rückflußkühler werden 13,33 g eines mehrfach mittelständigen Wasserstoffsiloxans (15,7 eq/kg SiH), 65,05 g $D_5$, 21,6 g eines Polymethylphenylsiloxans (500 cSt, ABCR) und 0,1 ml Trifluormethansulfonsäure vorgelegt und bei Raumtemperatur 24 Stunden gerührt. Nach vollständiger Equilibrierung wird durch Zugabe von 6,0 g Natriumhydrogencarbonat binnen 2 Stunden neutralisiert und nachfolgend filtriert. Es wurde ein farbloses, klares Silikonequilibrat erhalten.

S 3:

In einem Mehrhalskolben mit KPG-Blatt-Rührer, Rückflusskühler, Inertgaszuleitung und Temperaturfühler werden 14 g eines Siloxans der Formel $M_1$ $M^H{}_1$ $D_{123}$ $D^H{}_{25}T_0$ $Q_0$ ($R^1$ = Me) mit 69,94 g Allylpolyether 1, 2,98 g eines $M_{0,04}M^v{}_{1,96}$ $M^H{}_0$ $D_{147,1}$ $D^H{}_{0,9}$ $D^v{}_0$ $T_0$ $Q_0$ Siloxans (S1) sowie 20g Solvesso 150 innig miteinander vermischt und unter Inertgasatmosphäre durch Zugabe von 10 ppm Platin in Form eines Karstedt-Katalysators der trüben Reaktionsmischung die Hydrosilylierung initiiert. Dann wurde auf 80 - 90 °C Reaktionstemperatur erhitzt und die Exothermie wurde so abgefangen, dass die Reaktionstemperatur von 90°C nicht überschritten wurde. Nach 2,5 Stunden konnte kein freies SiH mehr nachgewiesen werden, der Umsatz war vollständig. Das leicht gelbe Produkt zeigte nach GPC-Analyse eine Molmassenverteilung von $M_n$ = 4744 g/mol und $M_w$ = 164457 ($M_w/M_n$ = 34,67) und eine Viskosität von 3,1 Pa s. Das so hergestellte Produkt wies bezogen auf die Reinsubstanz, d.h. ohne Lösungsmittel, einen Si-Gehalt von - 8 Gew. % auf.

S 4:

In einem Mehrhalskolben mit KPG-Blatt-Rührer, Rückflusskühler, Inertgaszuleitung und Temperaturfühler werden 16,1 g eines Siloxans der Formel $M_2$ $M^H{}_0$ $D_{67}$ $D^H{}_{24}T_0$ $Q_0$ (mit $R^1$ = Me oder Phenyl) welches in S2 hergestellt wurde mit 59,4 g Allylpolyether 1, 4,4 g eines $M_0$ $M^v{}_2$ $M^H{}_0$ $D_{350}$ $D^H{}_0$ $D^v{}_0$ To $Q_0$ (mit $R^1$ = Me und $R^3$ = $-CH_2CH_2$) Siloxans und sowie 20 g Solvesso 150 innig miteinander vermischt und unter Inertgasatmosphäre auf 80 - 90°C Reaktionstemperatur erhitzt. Nach Erreichen der Reaktionstemperatur wurde durch die Zugabe von 10 ppm Platin in Form eines Karstedt-Katalysators zur bis dahin trüben Reaktionsmischung wurde die Hydrosilylierung initiiert. Dabei wurde die Exothermie so abgefangen, dass die Reaktionstemperatur von 90°C nicht überschritten wurde. Nach 4,5 Stunden konnte kein freies SiH mehr gefunden werden, der Umsatz war vollständig. Das leicht gelbe Produkt zeigte nach GPC-Analyse (THF) eine Molmassenverteilung von $M_n$ = 4849 g/mol und $M_w$ = 78619 ($M_w/M_n$ = 16,21) und eine Viskosität von 8,9 Pa s. Das so hergestellte Produkt weist bezogen auf die Reinsubstanz, d.h. ohne Lösungsmittel, einen Si-Gehalt von - 10 Gew. % auf.

S 5:

In einem Mehrhalskolben mit KPG-Rührer, Rückflusskühler, Inertgaszuleitung und Temperaturfühler werden 16,1 g eines Siloxans der Formel $M_1$ $M^H{}_1$ $D_{123}$ $D^H{}_{25}T_0$ $Q_0$ (mit $R^1$=Me) mit 63,2 g Allylpolyether 1, 2,6 g eines $M_0$ $M^v{}_6$ $M^H{}_6$ $D_{173}$ $D^H{}_0$ $D^v{}_0$ $T_0$ $Q_5$ Siloxans (mit $R^1$ = Me und $R^3$ = $-CH_2CH_2$) und sowie 20 g Solvesso 150 innig miteinander vermischt und unter Inertgasatmosphäre auf 80°C Reaktionstemperatur erhitzt. Nach Erreichen der Reaktionstemperatur wurde durch die Zugabe von 10 ppm Platin in Form eines Karstedt-Katalysators zur bis dahin trüben Reaktionsmischung die Hydrosilylierung initiiert. Dabei wurde die Exothermie so abgefangen, dass die Reaktionstemperatur von 90°C nicht überschritten wurde. Nach 3 Stunden konnte kein freies SiH mehr gefunden werden, der Umsatz war vollständig. Das leicht gelbe Produkt zeigte nach GPC-Analyse eine Molmassenverteilung von $M_n$ = 3886 g/mol und $M_w$ = 414335 g/mol ($M_w/M_n$ = 106,63) und eine Viskosität von 13 Pa s. Das so hergestellte Produkt weist bezogen auf die Reinsubstanz, d.h. ohne Lösungsmittel, einen Si-Gehalt von - 8 % auf.

<u>Beispiel 2: Anwendungsbeispiel einer Zusammensetzung gemäß Beispiel 1 als Entschäumer</u>

Die erfindungsgemäßen Zusammensetzungen wurden nach dem Schaumtest ASTM D 892 überprüft. Die ASTM D 892 beschreibt den Versuchsaufbau und die Durchführung zur Bestimmung des Schäumungsverhaltens von Ölen. In diesem Test wurden 190 ml des Testöls in einem 1 l Messzylinder gebracht und auf Testtemperatur erwärmt. Es wurde bei gegebener Testtemperatur ein Luftstrom (65% Luftfeuchte) durch einen Diffusor in die Testflüssigkeit

eingeleitet (1013 mbar), siehe Fig 1, wobei Y den entstehenden Schaum bezeichnet. Nach 300 s wurde die Luftzufuhr abgestellt und das Schaumvolumen (SV) in Millilitern zwischen Luft- und Ölschicht gemessen. Gemessen wurde auch die Schaumzerfallszeit (SZ) in Sekunden, welche dann beendet ist, wenn der volle Flüssigkeitsspiegel sichtbar wird. Dieser Test wird nach ASTM D 892 gewöhnlich für Schmieröle bei Raumtemperatur und bei 93,5°C durchgeführt, mit einem Luftstrom von 94 ml/min. Zur Anpassung an die vorliegende Aufgabe wurde in einem zweiten Durchgang die Testtemperatur auf 150°C erhöht bei gleichzeitiger Erhöhung des Luftstroms auf 1000 ml/min, der erste Durchgang wurde bei einer Temperatur von 24°C und einem Luftstrom von 94 ml/min durchgeführt

Die erfindungsgemäßen Zusammensetzungen wurden mit organomodifizierten Siloxanen, wie diese für Getriebeöle Stand der Technik sind verglichen, ferner mit Polydimethylsiloxanen (Silikonölen) mit einer dynamischen Viskosität von 300 000 cSt und 2 000 000 cSt wie sie als Stand der Technik bei Motorenölen, Destillationen und in Crackprozessen eingesetzt werden und weiterhin mit Mischungen dieser Silikonöle mit Silikonharzen. Nach dem Stand der Technik ist es vorteilhaft den Entschäumer aus einem Lösungsmittel heraus zuzugeben. Für den gewählten Test wurden die Entschäumer zu 5 Gew.-% in einem aromatenhaltigen Lösungsmittel (Hydrosol A 200 ND) gelöst und zu dem zu entschäumenden Öl gegeben. Als Blindprobe wurde ein Vergleichsversuch ohne Anwesenheit eines Entschäumer verwendet. Als Kohlenwasserstoff wurde ein herkömmliches Paraffinöl ohne weitere Additive mit einer Viskosität bei 100 °C von 5,5 $mm^2/s$ verwendet. Dieses Öl ist als Basisöl mit der Bezeichnung SN 150 von Shell erhältlich.

Testbedingungen

Testapparatur: ASTM D 892
Testöl: SN 150 von Shell: Gruppe 1 Basisöl mit einer Viskosität von 5,5 $mm^2/s$ bei 100°C
Entschäumer: 20 ppm Entschäumer als 5 Gew.-% Lösung des Entschäumers in Hydrosol 200 ND.
Vergleichsentschäumer (nicht erfindungsgemäß):

- V1: Silikonöl DC 200 Fluid, 300 000 cSt, von Dow Coming
- V2: Silikonöl DC 200 Fluid, 2 000 000 cSt, von Dow Coming
- V3: Herkömmlicher kommerzieller Entschäumer für Kohlenwasserstoffe auf Basis organomodifizierter Siloxane von Evonik mit der Bezeichnung Tego Antifoam 2057
- V4: Herkömmlicher kommerzieller Entschäumer für Kohlenwasserstoffe auf Basis Polydimethyl-polyethersiloxan von Evonik mit der Bezeichnung Tegopren 5851
- V5: Mischung eines verzweigten Silikonharzes von Evonik mit der Bezeichnung Tegosivin HL 100, 5 Gew.-% gelöst in Silikonöl DC 200 Fluid mit 300.000 cSt

Als Blindprobe B1 wurde das Testöl SN 150 ohne Zusätze verwendet. Als zweite Blindprobe B2 wurde das Testöl mit einem Zusatz von 1 Vol.-% Hydrosol A 200 ND verwendet.

Tabelle 1: Schaumvolumen (SV) und Schaumzersetzungszeit (SZ) bei den angegeben Temperaturen gemäß Beispiel 2

| Beispiel | SV [ml], 150°C | SZ[s], 150°C | SV [ml], 24°C | SZ [s], 24°C |
|---|---|---|---|---|
| B1 | 800 | 43 | 420 | 280 |
| B2 | 850 | 44 | 400 | 270 |
| V1 | 420 | 24 | 10 | 5 |
| V2 | 300 | 18 | 10 | 5 |
| V3 | 450 | 30 | 10 | 5 |
| V4 | 600 | 35 | 10 | 5 |
| V5 | 250 | 15 | 10 | 5 |
| S3 | 200 | 10 | 10 | 1 |
| S4 | 300 | 10 | 20 | 1 |
| S5 | 250 | 14 | 20 | 1 |

Die Ergebnisse zeigen die Überlegenheit der verwendungsgemäß beanspruchten Zusammensetzungen gegenüber dem Stand der Technik. In den Experimenten bei Umgebungstemperatur zeichnen sich die verwendungsgemäßen Zusammensetzungen vorteilhaft durch eine niedrige Schaumzerfallszeit aus. In den Experimenten bei 150°C

zeichnen sich die verwendungsgemäßen Zusammensetzungen gegenüber den herkömmlichen organomodifizierten Polysiloxanen (V3 und V4) durch ein deutlich kleineres Schaumvolumen aus (Reduktion des Schaumvolumens um mindestens 30 Vol.%, bezogen auf den Stand der Technik)), weiterhin zeichnen sich die verwendungsgemäßen Zusammensetzungen durch ihre deutlich verringerte Schaumzerfallszeit aus.

Die verwendungsgemäß beanspruchten Verbindungen enthalten nur etwa 8-10% Si im Gegensatz zu den Vergleichsverbindungen.

Beispiel 2: Anwendungsbeispiel als Entschäumer in Prozessen oberhalb von 500°C

Schaumaufbau und Entschäumung unter Verkokungsbedingungen, welche die Bedingungen von Crackprozessen unter dem Einfluss von hohen Temperaturen in einem sogenannten Delayed Coker beschreiben, können in einer Glascokerapparatur nachvollzogen werden ("Foam Model for the Delayed Coker Pilot Unit", Pradipta Chattopadhyay, The University of Tulsa, Dissertation 2006).

Die Anlage zum Simulieren der Verkokungsprozesse ist eine 250 ml Destillationsapparatur mit Seitenarm (wassergekühlt) und Auffanggefäß der Zersetzungsprodukte. Der Heizmantel welcher den Glaskolben ca. zur Hälfte umschließt, kann ein von der Firma Aldrich (Glas-Col Series STM heating mantle) verwendetes Gerät sein, das eine Temperatur bis 650°C erzeugen kann. Nach Zugabe der zu entschäumenden Flüssigkeit werden 1 g Siedesteine zugegeben. Der Verschluss der Destillationsapparatur verfügt über 2 Öffnungen, eine zur Kontrolle der Temperatur mittels Digitalthermometer und die andere Öffnung ermöglicht die Anwendung einer Spritze, welche die Entschäumerlösung zudosiert.

Beim Aufheizprozess bildet sich je nach Testfluid bei unterschiedlichen Temperaturen ein Schaum aufgrund von Siede- bzw. Zersetzungsprozessen. Bei Begin der Schaumbildung wird die Entschäumerlösung zugegeben. Die Effektivität der Schaumunterdrückung wird untersucht, indem die Dauer bis zur nächsten Schaumbildung beobachtet und aufgezeichnet wird.

**Patentansprüche**

1. Verwendung von Zusammensetzungen zur Entschäumung von unter Anwendungsbedingungen flüssigen Kohlenwasserstoffen **dadurch gekennzeichnet, dass** Zusammensetzungen die Komponenten A und D enthalten, mit A enthaltend ein Polymer erhältlich durch Umsetzung im Sinne einer Hydrosilylierung von Verbindungen der Formel (I)

$$M_a M^v_b M^H_c D_d D^H_e D^V_f Tg Q_h \qquad \text{Formel (I)}$$

mit
$M = [R^1_3SiO_{1/2}]$
$M^V = [R^3R^1_2SiO_{1/2}]$
$M^H = [R^1_2SiHO_{1/2}]$
$D = [R^1_2SiO_{2/2}]$
$D^H = [R^1SiHO_{2/2}]$
$b^V = [R^3R^1SiO_{2/2}]$
$T = [R^1SiO_{3/2}]$
$Q = [SiO_{4/2}]$
a = 0 bis 42,
b = 0 bis 42,
c = 0 bis 42,
d = 5 bis 600,
e = 0 bis 50,
f = 0 bis 50,
g = 0 bis 20,
h = 0 bis 20,
mit der Maßgabe, dass folgende Bedingungen erfüllt sind
a + b + c größer oder gleich 2,
b + f größer 0,
c + e größer 0
und 0,24*(a+b+c+d+e+f+g) größer (c+e),
$R^1$ unabhängig voneinander gleiche oder verschiedene Alkylreste mit 1 bis 30 Kohlenstoffatomen, oder gleiche

oder verschiedene Arylreste mit 6 bis 30 Kohlenstoffatomen oder-OH oder-$OR^2$,
$R^2$ unabhängig voneinander gleiche oder verschiedene Alkylreste mit 1 bis 12 Kohlenstoffatomen, oder gleiche oder verschiedene Arylreste mit 6 bis 12 Kohlenstoffatomen,
$R^3$ unabhängig voneinander gleiche oder verschiedene organische Reste mit einer endständigen C-C-Doppelbindung oder einer terminalen oder inneren C-C-Dreifachbindung,
mit Verbindungen der Formel (I) und/oder mit anderen Verbindungen C, die eine C-C-Mehrfachbindung aufweisen, und nicht Formel (I) entsprechen,
und
D Metallatomen und/oder -ionen der Platingruppe, bevorzugt Platin-, Rhodium- und Rutheniumatomen, insbesondere Platinatomen.

2. Verwendung nach Anspruch 1 **dadurch gekennzeichnet dass**, die Komponente A der verwendeten Zusammensetzung ein Polymer ist erhältlich durch Umsetzung im Sinne einer Hydrosilylierung von Verbindungen der Formel (I) und Verbindungen der Formel (II)

$$M_i\, M^H_j\, D_k\, D^H_l\, T_m\, Q_n \qquad \text{Formel (II)}$$

mit
i = 0 bis 34,
j = 0 bis 34,
k = 5 bis 600,
l = 0 bis 50,
m = 0 bis 16,
n = 0 bis 16,
und den Bedingungen
i + j größer oder gleich 2 und
j + l größer oder gleich 2.

3. Verwendung nach den Ansprüchen 1 oder 2 **dadurch gekennzeichnet, dass** die Komponente A der verwendeten Zusammensetzung ein Polymer enthält, erhältlich durch Umsetzung im Sinne einer Hydrosilylierung von Verbindungen der Formel (I) mit einer oder mehreren ungesättigten Verbindungen C, und/oder dass die Komponente A ein Polymer enthält, erhältlich durch Umsetzung im Sinne einer Hydrosilylierung von Verbindungen der Formel (I) mit einer Verbindung der Formel (11) und einer oder mehreren ungesättigten Verbindungen C

4. Verwendung nach mindestens einem der Ansprüche 1 bis 3 **dadurch gekennzeichnet, dass** sie eine Komponente B enthält, erhältlich durch Umsetzung im Sinne einer Hydrosilylierung von Verbindungen der Formel (II), wie in Anspruch 2 definiert und ungesättigten Verbindungen C und/oder dass die verwendete Zusammensetzung eine oder mehrere Verbindungen C enthält.

5. Verwendung nach mindestens einem der Ansprüche 1 bis 4 **dadurch gekennzeichnet, dass** Verbindungen C der verwendeten Zusammensetzung Polyether der Formel (III) sind

$$CH_2{=}CHCH_2O(C_2H_4O)_o(C_2H_3(CH_3)O)_p(C_2H_3(C_2H_5)O)_q(C_2H_3(Ph)O)_rR^4 \qquad \text{Formel (III)}$$

mit
$R^4$ unabhängig voneinander gleiche oder verschiedene organische Reste, die keine der Hydrosilylierung zugängliche Mehrfachbindung tragen,
o = 0 bis 200,
p = 0 bis 200,
q = 0 oder größer 0 bis 100,
r = 0 oder größer 0 bis 100,
und den Bedingungen
o + p + q + r größer 3, o ist größer 0.

6. Verwendung nach mindestens einem der Ansprüche 1 bis 5 **dadurch gekennzeichnet, dass** die Zusammensetzung einen Siliziumgehalt von niedriger als 25 Gew. % aufweist, bezogen auf die Masse der Gesamtzusammensetzung.

7. Verwendung nach mindestens einem der Ansprüche 1 bis 6 **dadurch gekennzeichnet, dass** die Zusammensetzungen weitere Zusätze enthalten.

8. Verwendung nach einem der vorangegangenen Ansprüche **dadurch gekennzeichnet, dass** bei Destillationen und Vakuumdestillationen die Zusammensetzungen dem Destillationssumpf zugesetzt werden,
bevorzugt kontinuierlich zugesetzt werden.

9. Verwendung nach einem der vorangegangenen Ansprüche, **dadurch gekennzeichnet, dass** die Entschäumung in Prozessen bei Temperaturen von über 100 °C durchgeführt wird.

10. Verwendung nach einem der vorangegangenen Ansprüche, **dadurch gekennzeichnet, dass** in Crackprozessen, wie z. B. Delayed Coking, die Zusammensetzungen direkt auf den Schaum gesprüht werden.

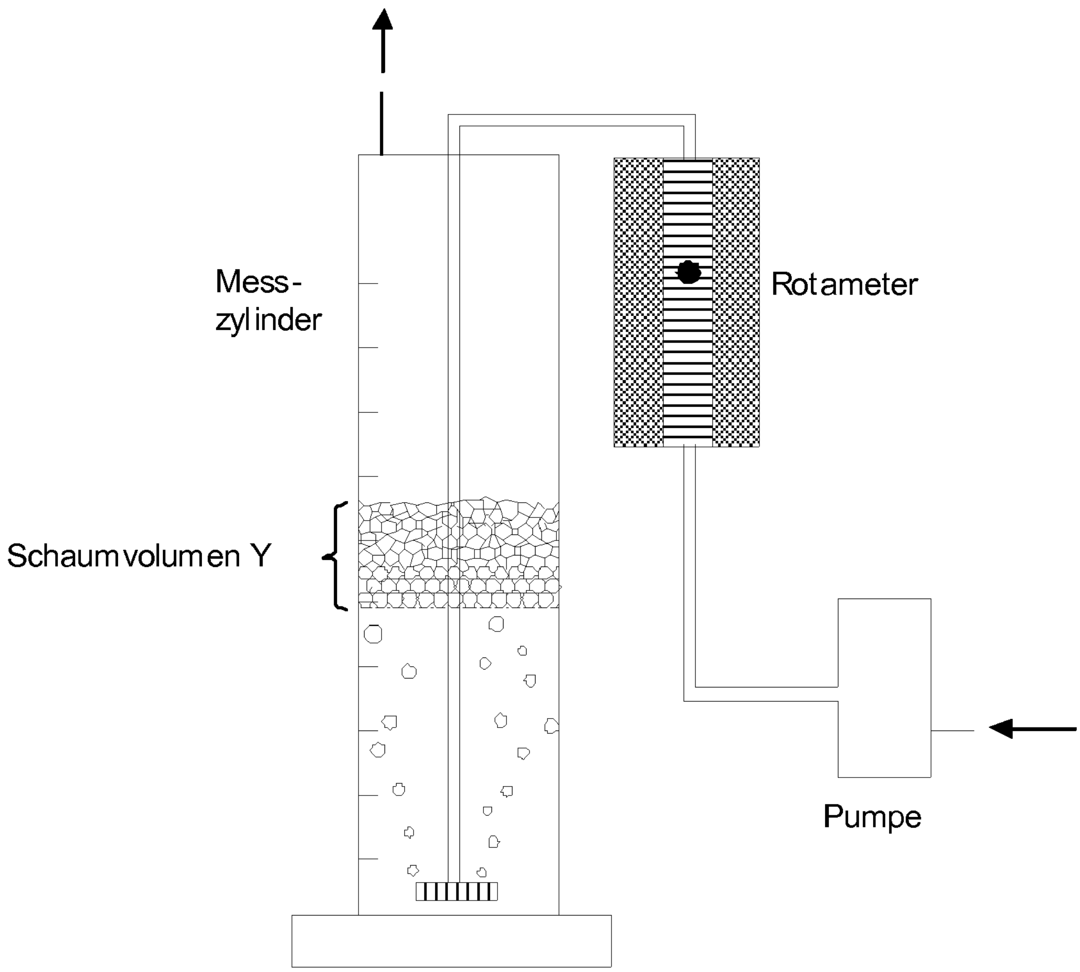


**Fig. 1**

Europäisches Patentamt
European Patent Office
Office européen des brevets

# EUROPÄISCHER RECHERCHENBERICHT

Nummer der Anmeldung
EP 13 15 3955

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 199 18 361 A1 (WACKER CHEMIE GMBH [DE]) 26. Oktober 2000 (2000-10-26)<br>* Seite 5, Zeile 49 - Zeile 50 *<br>* Seite 5, Zeile 52 *<br>* Seite 5, Zeile 55 *<br>* Seite 3, Zeile 15 - Zeile 37 *<br>* Seite 4, Zeile 58 - Zeile 61 *<br>----- | 1-5,7 | INV.<br>B01D19/04<br>C10M171/00<br>C10B55/00<br>C10G9/00 |
| A | DE 103 14 853 A1 (GOLDSCHMIDT AG TH [DE]) 14. Oktober 2004 (2004-10-14)<br>* Seite 5, Absatz 16 - Seite 6, Absatz 17 *<br>* Seite 6, Absatz 19 *<br>----- | 1,6,7 | |
| A | US 2005/109675 A1 (KREMER LAWRENCE N [US]) 26. Mai 2005 (2005-05-26)<br>* das ganze Dokument *<br>----- | 1-10 | |
| A | WO 2007/137948 A1 (DOW CORNING [US]; HILBERER ALAIN [FR]; NAVET EMILIE [BE]; VERMEIRE LAU) 6. Dezember 2007 (2007-12-06)<br>* Seite 5, Absatz 12 - Seite 7, Absatz 18 *<br>* Seite 8, Absatz 20 *<br>----- | 1-10 | RECHERCHIERTE SACHGEBIETE (IPC)<br>B01D<br>C10M<br>C10L<br>C10B<br>C10G<br>C08G |
| A | US 4 473 603 A (HOCKEMEYER FRIEDRICH [DE] ET AL) 25. September 1984 (1984-09-25)<br>* Spalte 3, Zeile 6 - Spalte 4, Zeile 68 *<br>----- | 1-10 | |

Der vorliegende Recherchenbericht wurde für alle Patentansprüche erstellt

| Recherchenort | Abschlußdatum der Recherche | Prüfer |
|---|---|---|
| Den Haag | 5. Juni 2013 | Hilgenga, Klaas |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

4
EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 13 15 3955

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-06-2013

| Im Recherchenbericht angeführtes Patentdokument | Datum der Veröffentlichung | Mitglied(er) der Patentfamilie | Datum der Veröffentlichung |
|---|---|---|---|
| DE 19918361 A1 | 26-10-2000 | KEINE | |
| DE 10314853 A1 | 14-10-2004 | KEINE | |
| US 2005109675 A1 | 26-05-2005 | EA 200600899 A1 | 29-12-2006 |
| | | US 2005109675 A1 | 26-05-2005 |
| | | WO 2005051512 A1 | 09-06-2005 |
| WO 2007137948 A1 | 06-12-2007 | BR PI0712727 A2 | 11-12-2012 |
| | | CA 2653516 A1 | 06-12-2007 |
| | | CN 101454061 A | 10-06-2009 |
| | | EP 2026891 A1 | 25-02-2009 |
| | | JP 2009538728 A | 12-11-2009 |
| | | RU 2008152381 A | 10-07-2010 |
| | | US 2009306282 A1 | 10-12-2009 |
| | | WO 2007137948 A1 | 06-12-2007 |
| | | ZA 200809530 A | 30-06-2010 |
| US 4473603 A | 25-09-1984 | DE 3115563 A1 | 04-11-1982 |
| | | EP 0063363 A1 | 27-10-1982 |
| | | FI 820218 A | 17-10-1982 |
| | | JP S57180655 A | 06-11-1982 |
| | | US 4473603 A | 25-09-1984 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DD 213945 A
- DE 4343235 C
- US 5613988 A
- US 5542690 A
- US 5334227 A
- US 20050109675 A
- US 4082690 A
- EP 1520870 A


### In der Beschreibung aufgeführte Nicht-Patentliteratur


- **DENKOV D.N.** Mechanismus of Action of Mixed Solid Liquid Antifoams-Stability of Oil Bridges in Foam Films. *Langmuir,* 1999
- **TOURRET ; WHITE.** Aeration and Foaming in Lubricating Oil Systems. *Aircraft Engng.,* 1952, vol. 24, 122-130137
- **VENZMER et al.** 5th International Colloquium Fuels. 12. Januar 2005, 483-488
- Foam Model for the Delayed Coker Pilot Unit. *Pradipta Chattopadhyay,* 2006